# EUROPÄISCHE PATENTSCHRIFT

(11) **EP 1 252 129 B1**
(45) Veröffentlichungstag und Bekanntmachung des Hinweises auf die Patenterteilung: **02.01.2004**
(21) Anmeldenummer: 01913764.5
(22) Anmeldetag: 24.01.2001
(51) Int. Cl.: C07C 51/43, C07C 57/07

(54) **REINIGUNGSVERFAHREN FÜR (METH)ACRYLSÄURE**
PURIFICATION METHOD FOR (METH)ACRYLIC ACID
PROCEDE DE PURIFICATION D'ACIDE (METH)ACRYLIQUE

(30) Priorität: 27.01.2000 DE 10003498
(43) Veröffentlichungstag der Anmeldung: 30.10.2002
(73) Patentinhaber: BASF AKTIENGESELLSCHAFT, 67056 Ludwigshafen (DE)
(72) Erfinder: DE DECKER, Emile, 2900 Schoten (BE); BASTIAENSEN, Erik, 2950 Kapellen (BE); BAUMANN, Dieter, 69190 Walldorf (DE); ECK, Bernd, 68519 Viernheim (DE); HEILEK, Jörg, 69245 Bammental (DE)
(74) Vertreter: Isenbruck, Günter, Dr.
(86) Internationale Anmeldenummer: PCT/EP2001/000743
(87) Internationale Veröffentlichungsnummer: WO 2001/055076

(56) Entgegenhaltungen:
- EP-A- 0 616 998
- DE-A- 10 003 497
- US-A- 5 831 124

## Beschreibung

Die Erfindung betrifft ein Reinigungsverfahren, das sowohl für Methacrylsäure als auch für Acrylsäure geeignet ist. Der Begriff (Meth)acrylsäure steht im folgenden für "Methacrylsäure oder Acrylsäure".

Acrylsäure ist eine bedeutende Grtmdchemikalie. Aufgrund ihrer sehr reaktionsfähigen Doppelbindung sowie der Säurefunktion eignet sie sich insbesondere als Monomeres zur Herstellung von Polymerisaten. Von der hergestellten Menge an Acrylsäuremonomeren wird der größere Teil vor der Polymerisation - zu z.B. Klebstoffen, Dispersionen oder Lacken - verestert. Nur der kleinere Teil der hergestellten Acrylsäuremonomeren wird direkt - zu z.B. "Superabsorbern" - polymerisiert. Während im allgemeinen bei der direkten Polymerisation der Acrylsäure Monomere hoher Reinheit benötigt werden, sind die Anforderungen an die Reinheit der Acrylsäure nicht so hoch, wenn'diese vor der Polymerisation verestert wird.

Es ist allgemein bekannt, daß Acrylsäure durch heterogen katalysierte Gasphasenoxidation von Propen mit molekularem Sauerstoff an im festen Aggregatzustand befindlichen Katalysatoren bei Temperaturen zwischen 200 und 400 °C zweistufig über Acrolein hergestellt werden kann. Hierbei werden oxidische Mehrkomponenten-Katalysatoren z.B. auf der Basis von Oxiden der Elemente Molybdän, Chrom, Vanadium oder Tellur eingesetzt.

Zur Aufarbeitung des bei der katalytischen Gasphasenoxidation anfallenden Gasgemisches sind mehrere Verfahren vorgeschlagen worden.

Die WO-A 9 801 415 offenbart ein Verfahren zur Herstellung von (Meth)acrylsäure, bei dem das durch katalytische Gasphasenoxidation erhaltene Gasgemisch kondensiert wird und (Meth)acrylsäure aus der dabei entstehenden wäßrigen Lösung ohne Zusatz von Hilfsstoffen auskristallisiert werden kann.

In EP-B 0 616 998 wird ein Verfahren zur Reinigung von Acrylsäure mittels fraktionierter Kristallisation beschrieben, wobei die Acrylsäure durch eine Kombination von dynamischer und statischer Kristallisation in mehreren Stufen mittels Kristallisations-/Schmelzzyklen gereinigt wird, und dabei der Rückstand der dynamischen Kristallisation durch die statische Kristallisation weiter eingeengt wird. Als dynamische Kristallisation werden entweder eine Fallfilmschichtkristallisation oder eine Suspensionskristallisation beschrieben.

Die Aufgabe der vorliegenden Erfindung bestand daher darin, ein Verfahren zur Reinigung von (Meth)acrylsäure zu schaffen, das für einen breiten Konzentrationsbereich der kristallisierenden Säuren und einen breiten Ausbeutebereich eine besonders wirtschaftliche Aufarbeitung ermöglicht.

Zur Lösung dieser Aufgabe wird die Kombination von wenigstens zwei verschiedenen dynamischen Kristallisationsverfahren vorgeschlagen.

Somit betrifft die Erfindung ein Verfahren zur Reinigung von (Meth)acrylsäure durch Kristallisation, das dadurch gekennzeichnet ist, daß die (Meth)acrylsäure durch eine Kombination von wenigstens zwei verschiedenen dynamischen Kristallisationsverfahren gereinigt wird. Bevorzugte Ausführungsformen der Erfindung sind in der nachfolgenden Beschreibung, den Figuren, dem Beispiel und den Unteransprüchen beschrieben.

In den Figuren zeigen:
- Fig. 1A und B: bevorzugte Verfahren mit Suspensions- und Schichtkristallisation;
- Fig. 2A und B: bevorzugte Verfahren mit zusätzlicher statischer Kristallisation und/oder Destillation;
- Fig. 3A: ein weiteres bevorzugtes Verfahren mit zwei Schichtkristallisationsstufen, einer Suspensionskristallisationsstufe und mit statischer Kristallisation und/oder Destillation;
- Fig. 3B: ein weiteres bevorzugtes Verfahren mit zwei Schichtkristallisationsstufen, zwei Suspensionskristallisationsstufen und einer Destillation; und
- Fig. 4: ein weiteres bevorzugtes Ausführungsbeispiel mit einer Suspensionskristallisationsstufe und fünf Schichtkristallisationsstufen.

Geeignete Gemische, aus denen das Ausgangsgemisch für das vorliegende Verfahren gewonnen werden kann, werden bevorzugt durch katalytische Gasphasenoxidation von C₃- bzw. C₄-Alkanen, -Alkenen, -Alkanolen und/oder -Alkanalen und/oder Vorstufen davon hergestellt. Besonders vorteilhaft wird das Gemisch durch katalytische Gasphasenoxidation von Propen, Acrolein, tert.-Butanol, Isobuten, Isobutan, Isobutyraldehyd, Methacrolein, Isobuttersäure oder Methyl-tert.butylether hergestellt. Als Ausgangsverbindungen können alle Vorstufen der genannten Verbindungen verwendet werden, bei denen sich die eigentliche C₃-/C₄-Ausgangsverbindung erst intermediär während der Gasphasenoxidation bildet. Beispielhaft genannt für die Herstellung der Methacrylsäure sei Methyl-tert.butylether oder Isobuttersäure.

Besonders vorteilhaft ist die katalytische Gasphasenreaktion von Propen und/oder Acrolein zu Acrylsäure mit molekularem Sauerstoff nach bekannten Verfahren, beispielsweise nach DE-A 196 24 31. Vorzugsweise wird hierbei bei Temperaturen zwischen 200 und 450°C und ggf. erhöhtem Druck gearbeitet. Vorzugsweise werden als heterogene Katalysatoren oxidische Mehrkomponenten-Katalysatoren auf der Basis der Oxide von Molybdän, Bismut und Eisen in der 1. Stufe (Oxidation von Propen zu Acrolein) und der Oxide von Molybdän und Vanadium in der 2. Stufe (Oxidation von Acrolein zu Acrylsäure) eingesetzt. Wird Propan als Ausgangsstoff verwendet, so kann dieses zu einem Propen-/Propan-Gemisch umgesetzt werden durch katalytische Oxidehydrierung, wie in US-A 5 510 558 beschrieben, durch homogene Oxidehydrierung, entsprechend CN-A 1 105 352 oder durch katalytische Dehydrierung, wie in EP-A 0 253 409 beschrieben. Bei Einsatz eines Propen-/Propan-Gemisches wirkt Propan als Verdünnungsgas. Geeignete Propen-/Propan-Gemische sind auch Raffineriepropen (70 % Propen und 30 % Propan) oder Crackerpropen (95 % Propen und 5 % Propan). Grundsätzlich können Propen-/Propan-Gemische wie die oben genannten mit Sauerstoff oder Luft oder einem Gemisch aus Sauerstoff und Stickstoff jeder Zusammensetzung zu Acrolein und Acrylsäure oxidiert werden.

Die Umsetzung von Propen zu Acrylsäure ist stark exotherm. Das Reaktionsgas, das neben den Edukten und Produkten vorteilhafterweise ein inertes Verdünnungsgas, z.B. Luftstickstoff, einen oder mehrere gesättigte C₁-C₆-Kohlenwasserstoffe, insbesondere Methan und/oder Propan und/oder Wasserdampf enthält, kann daher nur einen kleinen Teil der Reaktionswärme aufnehmen. Obwohl die Art der verwendeten Reaktoren an sich keiner Beschränkung unterliegt, werden meist Rohrbündelwärinetauscher verwendet, die mit dem Oxidationskatalysator gefüllt sind, da bei diesen der überwiegende Teil der bei der Reaktion freiwerdenden Wärme durch Konvektion und Strahlung an die gekühlten Rohrwände abgeführt werden kann.

Bei der katalytischen Gasphasenoxidation wird nicht reine Acrylsäure, sondern ein gasförmiges Gemisch erhalten, das neben der Acrylsäure als Nebenkomponenten im wesentlichen nicht umgesetztes Acrolein und/oder Propen, Wasserdampf, Kohlenmonoxid, Kohlendioxid, Stickstoff, Propan, Sauerstoff, Essigsäure, Propionsäure, Formaldehyd, weitere Aldehyde und Maleinsäureanhydrid enthalten kann. Üblicherweise enthält das Reaktionsproduktgemisch, jeweils bezogen auf das gesamte Reaktionsgemisch, 1 bis 30 Gew.-% Acrylsäure, 0,05 bis 1 Gew.-% Propen und 0,05 bis 1 Gew.-% Acrolein, 0,05 bis 10 Gew.-% Sauerstoff, 0,05 bis 2 Gew.-% Essigsäure, 0,01 bis 2 Gew.-% Propionsäure, 0,05 bis 1 Gew.-% Formaldehyd, 0,05 bis 2 Gew.-% Aldehyde, 0,01 bis 0,5 Gew.-% Maleinsäureanhydrid und 20 bis 98 Gew.-%, vorzugsweise 50 bis 98 Gew.-% inerte Verdünnungsgase. Als inerte Verdünnungsgase sind insbesondere gesättigte C₁-C₆-Kohlenwasserstoffe, wie 0 bis 90 Gew.-% Methan und/oder Propan, daneben 1 bis 30 Gew.-% Wasserdampf, 0,05 bis 15 Gew.-% Kohlenoxide und 0 bis 90 Gew.-% Stickstoff, jeweils bezogen auf 100 Gew.-% Verdünnungsgas, enthalten.

Die Methacrylsäure kann analog zu Acrylsäure durch katalytische Gasphasenreaktion von C₄-Ausgangsverbindungen mit molekularem Sauerstoff hergestellt werden. Besonders vorteilhaft ist die Methacrylsäure durch katalytische Gasphasenoxidation von Isobuten, Isobutan, tert.-Butanol, Isobutyraldehyd, Methacrolein oder Methyl-tert.-butylether erhältlich. Als Katalysatoren werden ebenfalls übergangsmetallische Mischoxidkatalysatoren (z. B. Mo, V, W und/oder Fe) verwendet. Besonders geeignete Verfahren sind solche, bei denen die Herstellung ausgehend von Methacrolein erfolgt, insbesondere dann, wenn das Methacrolein durch gasphasenkatalytische Oxidation von tert.-Butanol, Isobutan oder Isobuten oder durch Umsetzung von Formaldehyd mit Propionaldehyd gemäß EP-B-0 092 097 erzeugt wird. Somit besteht auch die Möglichkeit, Methacrylsäure zweistufig herzustellen durch (1) Kondensation von Propionaldehyd mit Formaldehyd (in Gegenwart eines sekundären Amins als Katalysator) zu Methacrolein und (2) anschließende Oxidation des Methacroleins zu Methacrylsäure.

Ebenso wie bei der Herstellung der Acrylsäure wird nicht reine Methacrylsäure, sondern ein gasförmiges Gemisch erhalten, das neben der Methacrylsäure als Nebenkomponenten im wesentlichen nicht umgesetztes Methacrolein und/oder Wasserdampf, Kohlenmonoxid, Kohlendioxid, Stickstoff, Sauerstoff, Essigsäure, Propionsäure, weitere Aldehyde und Maleinsäureanhydrid enthalten kann.

Zur Herstellung des Ausgangsgemisches für das vorliegende Verfahren kann das oben beschriebene Reaktionsproduktgemisch einer Kondensation, insbesondere einer Partial- oder Totalkondensation unterzogen werden, wobei eine Lösung erhalten wird. Die Kondensation kann nach üblichen Verfahren ein- oder mehrstufig erfolgen, wobei die Art der Kondensation keiner besonderen Beschränkung unterliegt. Vorteilhafterweise wird die Kondensation mit einem Direktkondensator durchgeführt, wobei bereits erzeugtes Kondensat mit dem heißen gasförmigen Reaktionsprodukt in Kontakt gebracht wird. Als Apparate für die Kondensation eignen sich insbesondere Sprühwäscher, Venturiwäscher, Blasensäulen oder Apparate mit berieselten Oberflächen.

Das die (Meth)acrylsäure enthaltende Ausgangsgemisch für das erfindungsgemäße Reinigungsverfahren unterliegt keinen besonderen Beschränkungen. Zweckmäßigerweise enthält es 75 bis 99,9 Gew.-% (Meth)acrylsäure, bevorzugt 90 bis 99,7 Gew.-% (Meth)acrylsäure, besonders bevorzugt 95 bis 99,5 Gew.-% (Meth)acrylsäure, jeweils bezogen auf 100 Gew.-% Ausgangsgemisch. Den Rest bilden Wasser sowie weitere Verunreinigungen, wie Säuren, Aldehyde, insbesondere Acrolein, Essigsäure, Propionsäure, Diacrylsäure, Maleinsäure(anhydrid) und der Polymerisationsinhibitor Phenothiazin.

Das Ausgangsgemisch wird erfindungsgemäß einer Kombination von mindestens zwei verschiedenen dynamischen Kristallisationsverfahren unterworfen, d.h. mindestens zwei Kristallisationsstufen. Bevorzugt werden die miteinander kombinierten Stufen der dynamischen Kristallisationen als fraktionierte Kristallisation ausgeführt. Üblicherweise werden bei einer fraktionierten Kristallisation alle Stufen oberhalb der Zuführung des Ausgangsgemisches, das heißt zu reineren Gemischen hin, Reinigungsstufen genannt und alle anderen Stufen, das heißt unterhalb der Zuführung des Ausgangsgemisches, Abtriebsstufen genannt. Zweckmäßigerweise werden mehrstufige Kristallisationsverfahren nach dem Gegenstromprinzip betrieben, wonach das Kristallisat jeder Stufe nach Abtrennung von der Mutterlauge der jeweiligen Stufe mit dem nächst höheren Reinheitsgrad zugeführt wird, während der Kristallisationsrückstand, d.h. die Mutterlauge, der jeweiligen Stufe mit dem nächst niedrigeren Reinheitsgrad zugeführt wird. Dabei werden üblicherweise die Kristallisationsstufen entsprechend dem Reinheitsgrad des jeweiligen Kristallisats als höhere bzw. niedrigere Kristallisationsstufen bezeichnet. Entsprechend wird die Abtriebsstufe, die das Kristallisat beziehungsweise die Mutterlauge mit dem niedrigsten Reinheitsgrad erzeugt, als unterste Kristallisationstufe bezeichnet, die Reinigungsstufe mit dem höchsten Reinheitsgrad als oberste Stufe bezeichnet.

Erfindungsgemäß unterliegt die Zahl der eingesetzten Kristallisationsstufen grundsätzlich keinen Einschränkungen. Insbesondere wird sie durch die Zusammensetzung des Ausgangsgemisches, die angestrebte Reinheit des Endproduktes und die angestrebte Ausbeute bestimmt. Vorteilhafterweise wird die fraktionierte Kristallisation mit wenigstens einer Abtriebsstufe und wenigstens einer Reinigungsstufe durchgeführt.

Bei den erfindungsgemäß eingesetzten dynamischen Kristallisationsverfahren handelt es sich grundsätzlich um jedes Kristallisationsverfahren, das unter erzwungener Bewegung der Mutterlauge durchgeführt wird. Erfindungsgemäß werden wenigstens zwei verschiedene dynamische Kristallisationsverfahren eingesetzt, wobei der Einsatz zweier verschiedener dynamischer Kristallisationsverfahren besonders bevorzugt ist. Die Art der eingesetzten dynamischen Kristallisationsverfahren unterliegt keiner besonderen Beschränkung. Geeignete Verfahren sind eine Suspensionskristallisation, eine Fallfilmschichtkristallisation, eine Schichtkristallisation vom Typ volldurchströmtes Rohr oder eine Schichtkristallisation auf bewegten Kühlflächen (Kühlband, Kühlwalze), wobei bevorzugt eine Kombination von Suspensionskristallisation und Fallfilmschichtkristallisation eingesetzt wird.

Das erfindungsgemäße Verfahren ist nicht beschränkt bezüglich der Verfahrensführung der eingesetzten dynamischen Kristallisationsverfahren. Diese können kontinuierlich oder diskontinuierlich sein. Sowohl eine Suspensionskristallisation als auch eine Schichtkristallisation können kontinuierlich oder diskontinuierlich betrieben werden. Vorzugsweise wird die Suspensionskristallisation und eine Schichtkristallisation auf bewegten Kühlflächen kontinuierlich durchgeführt, während die Fallfilmschichtkristallisation und die Schichtkristallisation vom Typ volldurchströmtes Rohr insbesondere diskontinuierlich betrieben wird. In einer weiteren bevorzugten Ausführungsform wird eine Suspensionskristallisation unterhalb der Schichtkristallisation durchgeführt. Besonders bevorzugt wird die Suspensionskristallisation im Abtriebsteil, insbesondere in allen Abtriebsstufen eingesetzt, während die Schichtkristallisation bevorzugt im Reinigungsteil, insbesondere in allen Reinigungsstufen eingesetzt wird.

Die Wänneabfuhrung bei den dynamischen Kristallisationsverfahren kann bevorzugt durch Kühlung von Apparatewänden oder durch Teilverdampfung der kristallisierenden Lösung im Vakuum erfolgen. Besonders bevorzugt wird die Wärmeabführung durch indirekte Kühlung über Wärmetauscherflächen bewirkt. Als Wärmeträger können alle hierzu geeigneten Mischungen eingesetzt werden, insbesondere Wasser/Methanol- oder Wasser/Glykol-Gemische.

Vorteilhafterweise liegt die Temperatur der Mutterlauge während der dynamischen Kristallisation zwischen -30 und +15°C, insbesondere zwischen -10 und +15°C, besonders bevorzugt zwischen -5 und +14°C.

Bei der Suspensionskristallisation handelt es sich um ein Kristallisationsverfahren, bei dem aus einem in der Regel feststofffreien, flüssigen Mehrkomponenten-System als Ausgangsmaterial, das in Lösung oder Schmelze vorliegt, durch Wärmeabführung in der Masse des Ausgangsmaterials Einzelkristalle gebildet werden. Die die Mutterlauge und die aus dispergierten Einzelkristallen bestehende feste Phase enthaltende Kristallsuspension muß während des Suspensionskristallisationsverfahrens bewegt werden, wozu insbesondere ein Umpumpen oder Rühren geeignet ist. Eine Haftung von Kristallen an Flächen ist hierbei nicht notwendig, sie ist sogar unerwünscht. Da die Kristallsuspension bewegt werden muß, wird die Suspensionskristallisation den dynamischen Kristallisationsverfahren zugerechnet.

Bei der Suspensionskristallisation durch indirekte Kühlung wird die Wärme über Kratzkühler, die mit einem Rührkessel oder einem Behälter ohne Rührwerk verbunden sind, abgeführt. Der Umlauf der Kristallsuspension wird hierbei durch eine Pumpe gewährleistet. Daneben besteht auch die Möglichkeit, die Wärme über die Wand eines Rührkessels mit wandgängigem Rührer abzuführen. Eine weitere bevorzugte Ausführungsform bei der Suspensionskristallisation ist die Verwendung von Kühlscheibenkristallisatoren, wie sie beispielsweise von der Firma GMF (Gouda in Holland) hergestellt werden. Bei einer weiteren geeigneten Variante zur Suspensionskristallisation durch Kühlung wird die Wärme über herkömmliche Wärmeüberträger (bevorzugt Rohrbündel- oder Plattenwänmeüberträger) abgeführt. Diese Apparate besitzen im Gegensatz zu Kratzkühlern, Rührkesseln mit wandgängigen Rührern oder Kühlscheiben keine Vorrichtung zur Vermeidung von Kristallschichten auf den wärmeübertragenden Flächen. Wird im Betrieb ein Zustand erreicht, bei dem der Wärmedurchgangswiderstand durch Verkrustung einen zu hohen Wert annimmt, erfolgt die Umschaltung auf einen zweiten Apparat. Während der Betriebszeit des zweiten Apparates wird der erste Apparat regeneriert (vorzugsweise durch Abschmelzen der Kristallschicht oder Durchspülen des Apparats mit ungesättigter Lösung). Wird im zweiten Apparat ein zu hoher Wärmedurchgangswiderstand erreicht, schaltet man wieder auf den ersten Apparat um, usw. Diese Variante kann auch mit mehr als zwei Apparaten im Wechsel betrieben werden. Außerdem kann die Kristallisation durch eine herkömmliche Verdampfung der Lösung im Vakuum erfolgen.

Für die Trennung des nach der dynamischen Kristallisation erhaltenen Fest-Flüssig-Gemisches eignen sich alle bekannten Verfahren der Fest-Flüssig-Trennung. Vorzugsweise werden die Kristalle durch Filtrieren, Sedimentieren und/oder Zentrifugieren von der Mutterlauge abgetrennt. Es ist jedoch auch möglich, die Mutterlauge vom dann bevorzugt ruhenden Kristallisat zum Beispiel durch Ablaufen lassen zu entfernen. Vorteilhafterweise wird dem Filtrieren, Sedimentieren oder Zentrifugieren eine Voreindickung der Suspension, z.B. durch Hydrozyklone, vorgeschaltet. Zum Zentrifugieren eignen sich alle bekannten Zentrifugen, die diskontinuierlich oder kontinuierlich arbeiten. Besonders vorteilhaft werden Schubzentrifugen verwendet, die ein- oder mehrstufig betrieben werden können. Daneben eignen sich auch Schneckensiebzentrifugen oder Schneckenaustragszentrifugen (Dekanter). Eine Filtration erfolgt vorteilhafterweise mittels Filternutschen, die kontinuierlich oder diskontinuierlich, mit oder ohne Rührwerk, oder mittels Bandfilter betrieben werden. Allgemein kann das Filtrieren unter Druck oder im Vakuum erfolgen. Während und/oder nach der Fest-Flüssig-Trennung können weitere Verfahrensschritte zur Steigerung der Reinheit der Kristalle bzw. des Kristallkuchens vorgesehen werden. In einer besonders vorteilhaften Ausgestaltung der Erfindung schließt sich nach dem Abtrennen der Kristalle von der Mutterlauge ein ein- oder mehrstufiges Waschen und/oder Schwitzen der Kristalle oder des Kristallkuchens an. Die verwendete Waschflüssigkeit unterliegt hierbei keiner Einschränkung. Vorteilhafterweise wird jedoch mit Reinware gewaschen, d.h. mit einer Flüssigkeit, die Säure enthält, deren Reinheit höher ist als die der Mutterlauge. Daneben ist auch eine Wäsche mit Wasser möglich. Das Waschen kann in hierfür üblichen Apparaten erfolgen, wie Waschkolonnen, in denen die Abtrennung der Mutterlauge und das Waschen in einem Apparat erfolgen, in Zentrifugen, die ein- oder mehrstufig betrieben werden können, oder in Filternutschen oder Bandfiltern. Das Waschen kann auf Zentrifugen oder Bandfiltern ein- oder mehrstufig durchgeführt werden, wobei die Waschflüssigkeit im Gegenstrom zum Kristallkuchen geführt werden kann. Daneben kann ein sogenanntes Schwitzen zur Erhöhung der Reinheit der Kristalle vorgesehen sein, bei dem es sich um ein lokales Abschmelzen verunreinigter Bereiche handelt. Besonders bevorzugt ist bei der Suspensionskzistallisation die Durchführung des Schwitzens auf Zentrifugen oder Bandfiltern, jedoch kann auch die Durchführung einer Kombination aus Waschen und Schwitzen in einem Apparat geeignet sein.

In besonders geeigneter Weise wird als Waschflüssigkeit für das Kristallisat einer gegebenen Kristallisationsstufe der Zulauf zu derselben Kristallisationstufe eingesetzt. Bevorzugt wird dabei das Massenverhältnis von Waschflüssigkeit zu Kristallisat im Bereich von 0,1 bis 1, besonders bevorzugt im Bereich von 0,2 bis 0,6 kg Waschflüssigkeit zu kg Kristallisat eingestellt.

Für die Durchführung der dynamischen Schichtkristallisation, bevorzugt einer Fallfilmschichtkrisiallisation oder einer Schichtkristallisation vom Typ volldurchströmtes Rohr, gibt es keine Beschränkungen. Die dynamische Schichtkristallisation an ruhenden Kühlflächen kann wie folgt durchgeführt werden. Die Kristalle der Säure werden dadurch auf die Kühlfläche aufgebracht, daß die Kühlfläche mit einem flüssigen Stoffgemisch, das die zu reinigende Säure enthält, in Kontakt gebracht wird und durch Abkühlung der Kühlfläche die entsprechenden Kristalle gebildet werden. Zur Bildung der Kristalle wird die Kühlfläche vorzugsweise in einem Temperaturbereich von der Lösetemperatur des jeweiligen flüssigen Stoffgemisches bis zu 60K darunter, vorzugsweise bis zu 40K darunter abgekühlt. Bei Erreichen der gewünschten Kristallmasse wird der Abkühlvorgang beendet. Danach kann die nicht kristallisierte, an der gewünschten Säure abgereicherte Restflüssigkeit abgeführt und somit von den Kühlflächen bzw. den gebildeten Kristallen entfernt werden. Das Abführen der Restflüssigkeit kann durch einfaches Abfließenlassen oder Abpumpen erfolgen.

Danach kann ein Wasch- und/oder Schwitzschritt durchgeführt werden. Beim Waschen werden die auf den Kühlflächen gewachsenen Kristalle mit einer Waschflüssigkeit in Kontakt gebracht und wieder von letzterer getrennt. Dadurch wird die auf den Kristallen verbleibende Restflüssigkeit durch die vorzugsweise reinere Waschflüssigkeit ausgetauscht. Insbesondere bei längerer Verweilzeit der Waschflüssigkeit auf den Kristallen erfolgt auch ein diffusiver Austausch von Verunreinigungen zwischen der reineren Waschflüssigkeit und weniger reinen Bereichen des Kristallisats. Als Waschflüssigkeit wird vorzugsweise frisches flüssiges Stoffgemisch, das die zu reinigende Säure enthält, oder Reinschmelze der Säure verwendet. Beim Schwitzen wird nach Abführen der Restflüssigkeit die Temperatur der Kristalle auf der Kühlfläche auf einen Wert angehoben, der zwischen der Gefrierpunkttemperatur der an der gewünschten Säure angereicherten Restflüssigkeit und der Schmelztemperatur der reinen Säure liegt. Das Schwitzen ist insbesondere dann vorteilhaft, wenn die Kristalle der Säure nicht als kompakte Kristallschicht vorliegen, sondern als poröses, einschlußreiches Haufwerk vorliegen. Danach können die Kristalle durch Erwärmung verflüssigt und die entstandene, an gewünschter Säure angereicherte Flüssigkeit abgeführt werden, was z.B. wiederum durch einfaches Abfließenlassen oder Abpumpen erfolgen kann. Die Verflüssigung der Kristalle erfolgt vorzugsweise in einem Temperaturbereich vom Schmelzpunkt der jeweiligen Säure bis 40K darüber, insbesondere bis 20K darüber.

In einer besonderen Ausgestaltung der dynamischen Schichtkristallisation wird auf den Kühlflächen als Restfilm nach dem Abschmelzen verbleibende, an Säure angereicherte Flüssigkeit als Impfkristallisat an der Kühlfläche partiell oder vollständig ausgefroren und danach erneut eine Kristallisation durchgeführt. Das Ausfrieren von Impfkristallisat kann auch dadurch erfolgen, daß vor der Kristallisation dadurch Impfkristallisat auf die Kühlfläche aufgebracht wird, indem die Kühlfläche in einem separaten Schritt mit einer, bezogen auf das zu trennende flüssige Stoffgemisch, reineren Schmelze, Lösung oder Suspension der Säure in Kontakt gebracht wird, anschließend davon abgetrennt wird und dann durch Abkühlen ein entsprechendes Impfkristallisat gebildet wird. Auch hierbei wird der auf den Kühlflächen verbleibende Restfilm durch Temperaturabsenkung an den Flächen partiell oder vollständig ausgefroren.

Die bei der dynamischen Schichtkristallisation verwendbaren Kühlflächen unterliegen an sich keiner Beschränkung und können beliebiger Form sein. Es können ein oder mehrere Kühlflächen verwendet werden. Vorzugsweise werden zylindrisch geformte Kühlflächen, z.B. Rohre, oder ebene Kühlflächen eingesetzt. Hierbei können die Kühlflächen entweder vollständig in die Flüssigkeit, aus der die gewünschte Säure zu reinigen ist, eingetaucht sein oder nur von einem Rieselfilm dieser Flüssigkeit überströmt werden, z.B. vollständig durchströmtes oder von einem Rieselfilm durch-/überströmtes Rohr. Die Kühlflächen können auch mit einem Zulauf und einem Ablauf versehene Teile eines Wärmetauschers sein.

Somit läßt sich eine besondere Ausführungsform der dynamischen Schichtkristallisation als diskontinuierlicher Prozeß mit folgendem Ablauf zusammenfassen:
1. Kühlfläche(n) und das flüssige Stoffgemisch, das die zu reinigende Säure enthält, werden in Kontakt gebracht;
2. die Kühlflächen werden abgekühlt, so daß Kristalle der Säure an den Flächen wachsen;
3. der Abkühlvorgang wird beendet bei Erreichen einer gewünschten Kristallmasse;
4. die nicht kristallisierte, an gewünschter Säure ärmere Restflüssigkeit wird von den Kühlflächen bzw. den gebildeten Kristallen entfernt;
5. die an den Kühlflächen befindlichen Kristalle werden durch Temperaturanhebung an den Flächen abgeschmolzen und die entstandene, an Säure angereicherte Flüssigkeit von den Flächen entfernt; und
6. auf den Flächen als Restfilm verbleibende, an Säure reichere Flüssigkeit kann als Impfkristallisat für den nächsten Kristallisierzyklus durch Temperaturabsenkung an den Flächen partiell oder vollständig ausgefroren werden.

Die dynamische Schichtkristallisation ist sowohl für die Kristallisation aus der Schmelze als auch aus der Lösung anwendbar. Besonders eignet sie sich zur Kristallisation aus der Schmelze.

Die Fallfilmschichtkristallisation kann beispielsweise wie in EP-B 0 616 998 beschrieben, durchgeführt werden.

In einer bevorzugten Ausführungsform des erfindungsgemäßen Reinigungsverfahrens wird die Kristallisation mit einer statischen Kristallisation und/oder einer Destillationsstufe kombiniert. Hierbei wird vorzugsweise mindestens ein Teil der Mutterlauge aus mindestens einer Abtriebsstufe der Kristallisation in einer statischen Kristallisation und/oder Destillation weiter aufgereinigt, wobei insbesondere die Mutterlauge aus der untersten dynamischen Kristallisationsstufe, bevorzugt einer Suspensionskristallisation, eingesetzt wird. Für die Durchführung der statischen Kristallisation gibt es keine Beschränkungen, beispielsweise kann wie in EP-B 0 616 998 beschrieben, verfahren werden. Bei Einsatz einer Destillationsstufe wird vorzugsweise ein Teil des sich bildenden Destillationsrückstands ausgeschleust und das sich bildende Kopfprodukt einer oder mehreren Abtriebsstufen der Kristallisation zugeftihrt, die unterhalb der Abtriebsstufe liegen, aus der die Mutterlauge für die Zuführung zur Destillationsstufe abgezogen wurde. Der Teil der Mutterlauge der mindestens einen Abtriebsstufe, der der Destillationsstufe zugeführt wird, beträgt vorzugsweise 5 bis 100 Gew.-%, insbesondere 50 bis 100 Gew.-%, besonders bevorzugt 90 bis 100 Gew.-% der jeweiligen Mutterlauge.

Für die Destillation kann grundsätzlich jede Destillationskolonne verwendet werden. Die Destillation, Destillationsstufe oder der Destillationsschritt kann einstufig oder mehrstufig durchgeführt werden. Bei einer mehrstufigen oder fraktionierten Destillation (auch Rektifikation genannt), die vorteilhafterweise in Rektifizierkolonnen durchgeführt wird, wird eine Kolonne mit Siebböden, z. B. Dualflowböden oder Querstfomsiebböden aus Metall, verwendet. Die Destillation kann auch mittels eines Verdampfers und eines nachgeschalteten Kondensators durchgeführt werden. Besonders bevorzugt sind hierbei Dünnschichtverdampfer als Fallstromverdampfer oder Dünnschichtverdampfer mit rotierenden Wischern. Als Kondensatoren werden die üblichen Kondensatoren eingesetzt, wobei diese keinen Beschränkungen unterliegen. Besonders bevorzugt sind Einspritzkondensatoren. Bei einer einstufigen Destillation wird zweckmäßigerweise ein einfacher Verdampfer, zum Beispiel eine Blase, und ein üblicher Kondensator eingesetzt.

Die Erfindung wird anhand der Zeichnungen näher erläutert, die bevorzugte Ausfühnmgsformen der Erfindung darstellen. Hierbei bezeichnen gleiche Bezugszeichen bzw. Bezugsziffern das gleiche.

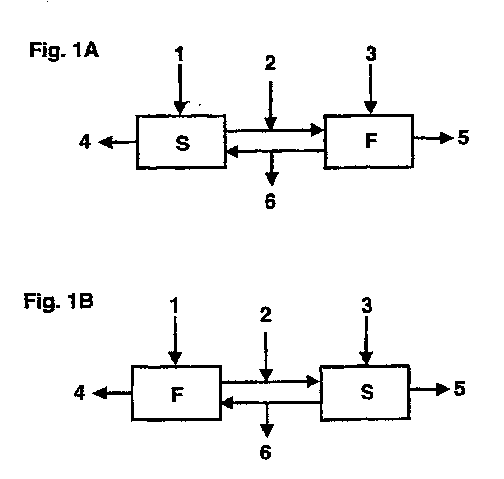

Fig. 1A zeigt ein Ausführungsbeispiel einer Kristallisation, die aus einer Suspensionskristallisation S und einer Schichtkristallisation F besteht, die beide ein- oder mehrstufig und kontinuierlich oder diskontinuierlich durchgeführt werden können. Hierbei ist die Suspensionskristallisation S unterhalb der Schichtkristallisation F angeordnet. Die Bezugsziffem 1, 2 und 3 bezeichnen mögliche Zuführungen für das aufzureinigende Ausgangsgemisch. Aus der Suspensionskristallisation S wird der Rückstand 4 abgeführt, während aus der Schichtkristallisation F das Reinprodukt 5 der gewünschten Säure abgeführt wird. Fig. 1B unterscheidet sich von Fig. 1A darin, daß die Schichtkristallisation unterhalb der Suspensionskristallisation angeordnet ist. Die Bezugsziffer 6 bezeichnet eine mögliche teilweise oder vollständige Ausschleusung der Mutterlauge bzw. des Rückstands der oberen dynamischen Kristallisation.

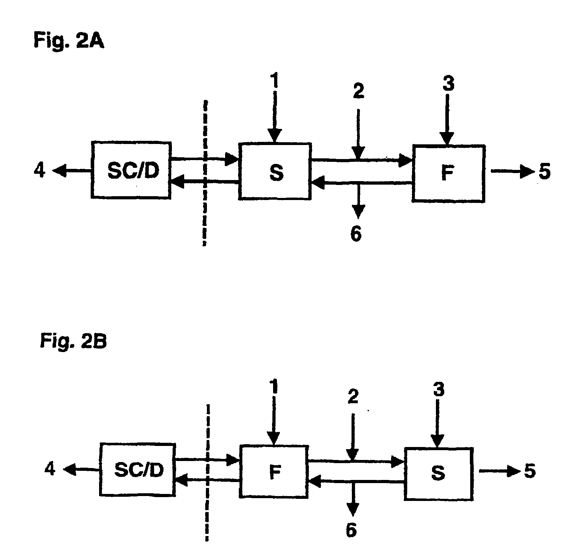

Fig. 2A und Fig. 2B zeigen die Ausführungsformen von Fig. 1A bzw. 1B, die um eine statische Kristallisation SC und/oder eine Destillationsstufe D ergänzt sind. In diesem Fall wird der Rückstand 4 aus dem statischen Kristallisationsteil und/oder dem Destillationsteil abgeführt.

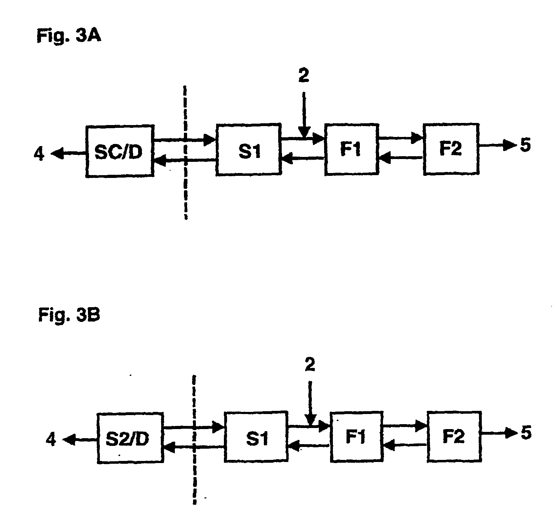

Fig. 3A zeigt eine bevorzugte Verschaltung des erfindungsgemäßen Verfahrens mit einer einstufigen kontinuierlichen Suspensionskristallisation S1 als Abtriebsstufe und zwei diskontinuierlichen Fallfilmkristallisationsstufen F1 und F2 als Reinigungsstufen. Der Rückstand der Suspensionskristallisation S1 wird einer statischen Kristallisation SC und/oder einer Destillationsstufe D zugeführt.

Fig. 3B zeigt die gleiche Kombination der dynamischen Kristallisationsstufen S1, F1 und F2 wie Fig. 3A, wobei der Rückstand der Stufe S1 einer Kombination von Destillation und einer weiteren Suspensionskristallisationsstufe S2 zugeführt wird, wobei bevorzugt die Suspensionskristallisationsstufe S2 unterhalb der Destillationsstufe D angeordnet ist.

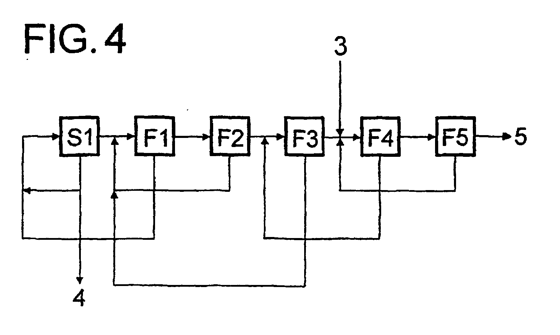

Fig. 4 zeigt als Ausführungsbeispiel der Erfindung die fraktionierte Kristallisation einer Acrylsäuremischung, wobei insgesamt 6 Kristallisationsstufen zum Einsatz kommen. Die Stufe S1 ist eine einstufige kontinuierliche Suspensionskristallisation, alle weiteren Stufen sind diskontinuierliche Fallfilmschichtkristallisationsstufen, wie beispielsweise in EP-B 0 616 998 beschrieben. Die Fallfilmstufen F1 bis F3 sind wie die Stufe S1 Abtriebsstufen der Kristallisation, die Stufen F4 und F5 sind Reinigungsstufen der Kristallisation.

Das erfindungsgemäße Verfahren ermöglicht durch die Kombination mindestens zweier verschiedener dynamischer Kristallisationverfahren für einen breiten Konzentrationsbereich der Ausgangssäure die Gewinnung einer reinen Säure mit hoher Ausbeute auf sehr wirtschaftliche Weise. Durch die erfindungsgemäße, geeignete Kombination dynamischer Verfahren können aufwendige Kristallisationsstufen eingespart und der Kristallisationsaufwand reduziert werden. Besonders gegenüber einer statischen Kristallisation haben die dynamischen Kristallisationsverfahren den Vorteil einer kürzeren Verweilzeit und einer besseren Reinigungswirkung pro Kristallisationsstufe. Das Trennergebnis ist wirtschaftlich gesehen dann besonders gut, wenn die Suspensionskristallisation unterhalb der Fallfilmschichtkristallisation angeordnet wird.

Das erfindungsgemäße Verfahren wird weiterhin anhand des folgenden Beispiels näher erläutert, das eine bevorzugte Ausführungsform der Erfindung darstellt.

### Beispiel

Ein Strom gemäß der in Tabelle 1 angegebenen Ausgangszusammensetzung wurde einer sechsstufigen fraktionierten Kristallisation mit einem Verschaltungsschema wie in Fig. 4 dargestellt, unterzogen. Nach Durchlaufen der Reinigungsstufen F4 und F5 wurde ein Reinprodukt mit der in Tabelle 1 angegebenen Zusammensetzung erhalten. Nach Durchlaufen der Abtriebsstufen F3 bis S1 wurde ein Rückstand mit der in Tabelle 1 angegebenen Zusammensetzung erhalten.

**Tabelle 1**

| | Ausgangsgemisch (Strom 3) | Reinprodukt (Strom 5) | Rückstand (Strom 4) |
|---|---|---|---|
| Acrylsäure | 99,45 Gew.-% | 99,97 Gew.-% | 52,96 Gew.-% |
| Essigsäure | 960 ppm | 147 ppm | 7,22 Gew.-% |
| Propionsäure | 330 ppm | 88 ppm | 2,15 Gew.-% |
| Diacrylsäure | 3100 ppm | 47 ppm | 27,46 Gew.-% |
| Wasser | 190 ppm | 23 ppm | 1,67 Gew.-% |
| Phenothiazin | 290 ppm | < 1 ppm | 2,57 Gew.-% |
| Furan-II-aldehyd | 220 ppm | < 1 ppm | 1,95 Gew.-% |
| Sonstige | 410 ppm | < 5 ppm | 4,02 Gew.-% |

Die Suspensionslaistallisationsstufe S1 erhielt als Zulauf die von der Stufe F 1 kommende Mutterlauge mit der in Tabelle 2 angegebenen Zusammensetzung. Das in der Stufe S1 erhaltene Kristallisat wies die in Tabelle 2 angegebene Zusammensetzung auf und wurde der Stufe F1 zugeführt. Die in der Stufe S1 nach Abtrennen der Kristalle erhaltene Mutterlauge wurde teilweise als Rückstand (Strom 4) ausgeschleust und wies die in Tabelle 1 gezeigte Zusammensetzung des Rückstandes auf. Der nicht ausgeschleuste Teil der Mutterlauge wurde in die Kristallisation zurückgeführt. Das Massenverhältnis von ausgeschleuster zu zurückgeführter Mutterlauge lag bei 1 : 8. Die Suspensionskristallisationsstufe S1 wurde in einem Rührkessel mit wandgängigem Rührer durchgeführt. Die Kristallisationstemperatur lag bei -8°C. Die Verweilzeit der Suspensionskristallisation lag bei 4 h. Die Abtrennung der erzeugten Kristalle erfolgte auf einer Siebzentrifuge bei einer Verweilzeit von 1 min auf der Zentrifuge (Siebkorbdurchmesser 200 mm, 2000 Umdrehungen/min). Es erfolgte keine Waschung des Filterkuchens.

**Tabelle 2**

| | Zulauf von F1 zu S1 | Kristallisat S 1 |
|---|---|---|
| Acrylsäure | 85,96 Gew.-% | 93,45 Gew.-% |
| Essigsäure | 2,92 Gew.-% | 1,95 Gew.-% |
| Propionsäure | 1,05 Gew.-% | 0,8 Gew.-% |
| Diacrylsäure | 7,32 Gew.-% | 2,75 Gew.-% |
| Wasser | 0,47 Gew.-% | 0,2 Gew.-% |
| Phenothiazin | 0,68 Gew.-% | 0,26 Gew.-% |
| Furan-II-aldehyd | 0,52 Gew.-% | 0,19 Gew.- |
| Sonstige | 1,08 Gew.-% | 0,4 Gew.-% |

Die mit der fraktionierten Kristallisation erreichte Ausbeute an Acrylsäure lag bei 99,4 %.

Das Beispiel zeigt, daß mittels der Kombination zweier verschiedener dynamischer Kristallisationsverfahren, nämlich einer Suspensionskristallisation mit Fallfilmschichtkristallisation, die Reinigung von Acrylsäure mit hoher Produktreinheit und insbesondere mit hoher Ausbeute des Reinigungsverfahrens möglich ist. Die Aufkonzentrierung von Verunreinigungen im Rückstand (= hohe Ausbeute) ist selbst ohne Waschen oder Schwitzen des Kristallisats in der untersten Stufe möglich.

Gegenüber einer Verweilzeit in der untersten Kristallisationsstufe nach dem Verfahren der Erfindung von 4 h beträgt die Verweilzeit im Kristallisator in jeder Stufe der statischen Kristallisation während des Kristallisierens und Schwitzens nach dem Stand der Technik gemäß EP-A 0 616 998 dagegen 8 bis 9 h. Somit wird erfindungsgemäß die Verweilzeit und, durch Einsparung einer Abtriebsstufe, der Kristallisieraufwand reduziert.

## Patentansprüche

1. Verfahren zur Reinigung von (Meth)acrylsäure durch Kristallisation, **dadurch gekennzeichnet, daß** die (Meth)acrylsäure durch eine Kombination von wenigstens zwei verschiedenen dynamischen Kristallisationsverfahren gereinigt wird.

2. Verfahren nach Anspruch 1, **dadurch gekennzeichnet, daß** die Kristallisation als fraktionierte Kristallisation durchgeführt wird.

3. Verfahren nach Anspruch 1 oder 2, **dadurch gekennzeichnet, daß** zwei verschiedene dynamische Kristallisationsverfahren eingesetzt werden.

4. Verfahren nach einem der Ansprüche 1 bis 3, **dadurch gekennzeichnet, daß** als dynamische Kristallisationsverfahren eine Suspensionskristallisation, die bevorzugt kontinuierlich durchgeführt wird, und eine Schichtkristallisation, die bevorzugt diskontinuierlich durchgeführt wird, eingesetzt werden.

5. Verfahren nach einem der vorhergehenden Ansprüche, **dadurch gekennzeichnet, daß** die Kristallisation mit wenigstens einer Abtriebstufe und wenigstens einer Reinigungsstufe durchgeführt wird.

6. Verfahren nach Anspruch 4 oder 5, **dadurch gekennzeichnet, daß** die Suspensionskristallisation unterhalb der Schichtkristallisation durchgeführt wird.

7. Verfahren nach Anspruch 5 oder 6, **dadurch gekennzeichnet, daß** alle Abtriebsstufen als Suspensionskristallisation und alle Reinigungsstufen als Schichtkristallisation durchgeführt werden.

8. Verfahren nach einem der Ansprüche 4 bis 7, **dadurch gekennzeichnet, daß** die Schichtkristallisation als Fallfilmschichtkristallisation durchgeführt wird.

9. Verfahren nach einem der vorhergehenden Ansprüche, **dadurch gekennzeichnet, daß** ein Ausgangsgemisch mit 75 bis 99,9 Gew.-% (Meth)acrylsäure, bezogen auf 100 Gew.-% Ausgangsgemisch, eingesetzt wird.

10. Verfahren nach einem der vorhergehenden Ansprüche, **dadurch gekennzeichnet, daß** die Kombination mindestens zweier verschiedener dynamischer Kristallisationsverfahren mit einer statischen Kristallisation und/oder einer Destillation kombiniert wird.

## Claims

1. A process for purifying (meth)acrylic acid by crystallization, wherein the (meth)acrylic acid is purified by a combination of at least two different dynamic crystallization processes.

2. A process as claimed in claim 1, wherein the crystallization is carried out as a fractional crystallization.

3. A process as claimed in claim 1 or 2, wherein two different dynamic crystallization processes are used.

4. A process as claimed in any of claims 1 to 3, wherein the dynamic crystallization processes used are a suspension crystallization which is preferably carried out continuously and a layer crystallization which is preferably carried out batchwise.

5. A process as claimed in any of the preceding claims, wherein the crystallization is carried out using at least one stripping stage and at least one purification stage.

6. A process as claimed in claim 4 or 5, wherein the suspension crystallization is carried out below the layer crystallization.

7. A process as claimed in claim 5 or 6, wherein all stripping stages are carried out as a suspension crystallization and all purification stages as a layer crystallization.

8. A process as claimed in any of claims 4 to 7, wherein the layer crystallization is carried out as a falling-film layer crystallization.

9. A process as claimed in any of the preceding claims, wherein a starting mixture comprising from 75 to 99.9% by weight, based on 100% by weight of starting mixture, of (meth)acrylic acid is used.

10. A process as claimed in any of the preceding claims, wherein the combination of at least two different dynamic crystallization processes is combined with a static crystallization and/or a distillation.

## Revendications

1. Procédé de purification d'acide (méth)acrylique par cristallisation, **caractérisé en ce que** l'acide (méth)acrylique est purifié par une combinaison d'au moins deux procédés différents de cristallisation dynamique.

2. Procédé selon la revendication 1, **caractérisée en ce que** la cristallisation est effectuée en mode de cristallisation fractionnée.

3. Procédé selon la revendication 1 ou 2, **caractérisé en ce que** l'on utilise deux procédés différents de cristallisation dynamique.

4. Procédé selon l'une quelconque des revendications 1 à 3, **caractérisé en ce que** l'on utilise comme procédés de cristallisation dynamique une cristallisation en suspension, qui est de préférence entreprise en continu et une cristallisation en couches, qui est de préférence entreprise en mode discontinu.

5. Procédé selon l'une quelconque des revendications qui précèdent, **caractérisé en ce que** la cristallisation est entreprise avec au moins une étape de stripage et au moins une étape de purification.

6. Procédé selon la revendication 4 ou 5, **caractérisé en ce que** la cristallisation en suspension est entreprise sous la cristallisation en couches.

7. Procédé selon la revendication 5 ou 6, **caractérisé en ce que** toutes les étapes de stripage sont entreprises en tant que cristallisation en suspension, et toutes les étapes de purification en tant que cristallisation en couches.

8. Procédé selon l'une quelconque des revendications 4 à 7, **caractérisé en ce que** la cristallisation en couches est entreprise en tant que cristallisation à film descendant.

9. Procédé selon l'une quelconque des revendications qui précèdent, **caractérisé en ce que** l'on met en oeuvre un mélange de départ comportant de 75 à 99,9% en poids d'acide (méth)acrylique, par rapport à 100% en poids de mélange de départ.

10. Procédé selon l'une quelconque des revendications qui précèdent, **caractérisé en ce que** la combinaison d'au moins deux procédés différents de cristallisation dynamique est combinée à une cristallisation statique et/ou à une distillation.
